# EUROPEAN PATENT SPECIFICATION

(11) **EP 0 375 718 B1**
(45) Date of publication and mention of the grant of the patent: **09.11.1994**
(21) Application number: 88907646.9
(22) Date of filing: 26.08.1988
(51) Int. Cl.: C12N 15/81, C12P 21/02, C12N 1/19

(54) **PROCESS FOR THE PRODUCTION OF APROTININ AND APROTININ HOMOLOGUES IN YEAST**
VERFAHREN ZUR HERSTELLUNG VON APROTININ UND APROTININ-HOMOLOGEN IN HEFE
PROCEDE DE PRODUCTION D'APROTININE ET DE SES HOMOLOGUES DANS LA LEVURE

(30) Priority: 28.08.1987 DK 4501/87
(43) Date of publication of application: 04.07.1990
(73) Proprietor: NOVO NORDISK A/S, 2880 Bagsvaerd (DK)
(72) Inventor: NORRIS, Kjeld, DK-2900 Hellerup (DK); NORRIS, Fanny, DK-2900 Hellerup (DK); Bjorn, Soren Erik, DK-2800 Lyngby (DK)
(74) Representative: Thalsoe-Madsen, Kine Birgit
(86) International application number: DK8800138
(87) International publication number: WO8901968

(56) References cited:
- EP-A- 0 163 529
- EP-A- 0 200 451
- EP-A- 0 419 878
- WO90/10075
- The Journal of Biological Chemistry, vol. 261, no.16, Issue of June 5, pp 7115-7118, 1986 (Cara Berman et al)
- The Embo Journal, vol. 5, no. 12, pp. 3219-3225, 1986 (Brigitte von Wilken-Bergmann et al.
- Chemical Abstracts, vol. 105 (1986) abstract no. 55328e, Microbiology (Washington, D.C.) 1986, 273-8 (Eng)
- Cell, vol. 32, 839-852, March 1983, (David Julius etal.

## Description

### FIELD OF THE INVENTION

The present invention relates to a process for the production of aprotinin and homologues thereof in yeast, synthetic genes encoding such products, expression vectors and transformed yeast cells.

### BACKGROUND OF THE INVENTION

Aprotinin (bovine pancreatic trypsin inhibitor, BPTI) is a polypeptide secreted by several bovine organs and tissues, such as lymph nodes, pancreas, lung, parotid gland, spleen and liver. It is a single chain polypeptide consisting of 58 amino acid residues cross-linked by three disulphide bridges having the following sequence: 
-
1   2   3   4   5   6   7   8   9   10  11  12  13  14
-
15  16  17  18  19  20  21  22  23  24  25  26  27  28
-
29  30  31  32  33  34  35  36  37  38  39  40  41  42
-
43  44  45  46  47  48  49  50  51  52  53  54  55  56

57  58
The three disulphide bridges are situated between Cys(5)-Cys(55), Cys(14)-Cys(38) and Cys(30)-Cys(51), respectively.

Aprotinin is used for the treatment of acute pancreatitis, various states of shock syndrome, hyperfibrinolytic haemorrhage, and myocardial infarction. Administration of aprotinin in high doses significantly reduces blood loss in connection with cardiac surgery.

Aprotinin can be extracted from various bovine organs or tissues, such as lung, pancreas and parotid glands. In the present specification the aprotinin thus obtained is referred to as native aprotinin. Extraction from animal tissues is a cumbersome process and requires large amounts of the bovine organ or tissue. A far more convenient method for commercial production would be a fermentation process by which a gene coding for aprotinin is incorporated in a suitable microorganism by means of recombinant DNA techniques and the microorganism is cultured in a suitable nutrient medium to produce the desired product which it secretes into the medium in mature form.

A gene for aprotinin has been fused to the coding sequence for E. coli alkaline phosphatase signal peptide and expressed in E. coli under the control of the alkaline phosphatase promoter (Marks, C.B. et al., The Journal of Biological Chemistry 261 (1986) 7115-7118). Also, a synthetic gene encoding the protein sequence of Met-aprotinin has been cloned in an E. coli expression vector (von Wilcken-Bermann, B. et al., The EMBO Journal 5 (1986) 3219-3225). Production of aprotinin and aprotinin homologues is also described in European published patent specification No. 238,993 and in British patent application No. 2,188,933.

However, a problem with the production of small foreign proteins in E. coli is the fact that such proteins are liable to be degraded by the host proteases Also, establishing of correct disulphide bridges and correct folding might be a problem in E. coli.

It has been shown that yeasts are capable of expressing and maturing small proteins of about the same size as aprotinin. In EP-patent application No. 0163529A production of insulin precursors with correctly positioned disulphide bridges is described, and in EP-patent application No. 0189998A production of glucagon, a single-chain polypeptide of 29 amino acid residues is described.

By these methods a primary product consisting of the desired protein linked to a yeast leader peptide is expressed in yeast. During secretion the primary product is processed by means of the Kex2 enzyme (Julius, D. et al. Cell 32 (1983) 839-852) and the processed product, which is also referred to as the mature or maturated product, is then secreted into the medium. Processing of the expressed primary product occurs at a pair of basic amino acids (Lys-Arg) at the N-terminal end of the mature product.

In the case of aprotinin, which has a basic amino acid (Arg) as the N-terminal amino acid, problems might arise if the Kex2 processing site Lys-Arg is inserted before the first Arg residue of the aprotinin sequence since a "triple basic" cleavage site would be present in the expressed fusion product. Also, the pair of basic amino acids in the aprotinin molecule (Lys(41)-Arg(42)) might be a potential cleavage site for the Kex2 enzyme within the yeast cells.

### SUMMARY OF THE INVENTION

It is the purpose of the present invention to provide a method for the production of aprotinin or homologues thereof in yeast by which high amounts of correctly folded and maturated protein is secreted to the medium.

The invention is based on the surprising discovery that aprotinin and certain homologues thereof can be produced in high yields with correctly positioned disulphide bridges by cultivation of a yeast strain transformed with a DNA-sequence encoding such products.

According to a first aspect of the present invention there is provided a method for producing high yields of aprotinin or homologues thereof in yeast by cultivation of a yeast strain containing a replicable expression vector containing a synthetic gene encoding aprotinin or homologues thereof fused to a DNA sequence encoding a signal and leader peptide providing for secretion of the aprotinin or aprotinin homologue, in a suitable nutrient medium followed by recovery of the aprotinin or aprotinin homologue from the culture medium.

According to a second aspect of the present invention there is provided a replicable expression vector comprising a DNA sequence encoding aprotinin or an aprotinin homologue fused to a DNA sequence encoding a signal and leader peptide providing for secretion of the aprotinin or aprotinin homologue, that allows the expression of the aprotinin or its homologue in yeast.

According to a third aspect of the present invention there is provided a yeast strain transformed with this replicable expression vector.

The aprotinins produced by the present invention can be characterized by the following formula (I)

X-aprotinin(3-40)-Y-Z-aprotinin(43-58) (I)

in which X means Arg-Pro, Pro or hydrogen, aprotinin(3-40) means the amino acid sequence from amino acid residue 3 to 40 in native aprotinin, Y is Lys, Z may be Arg or Ser, and aprotinin(43-58) means the amino acid sequence from amino acid residue 43 to 58 in native aprotinin.

### BRIEF DESCRIPTION OF THE DRAWINGS

The present invention will be further illustrated by reference to the accompanying drawings in which:
Fig. 1 shows a synthetic gene encoding aprotinin(3-58);
Fig. 2 illustrates the construction of plasmids pKFN374 and pKFN375;
Fig. 3 illustrates the construction of plasmid pMT 636;
Fig. 4 shows a synthetic gene encoding aprotinin(3-58,42 Ser);
Fig. 5. Illustrates the construction of plasmids pKFN414 and pKFN416;

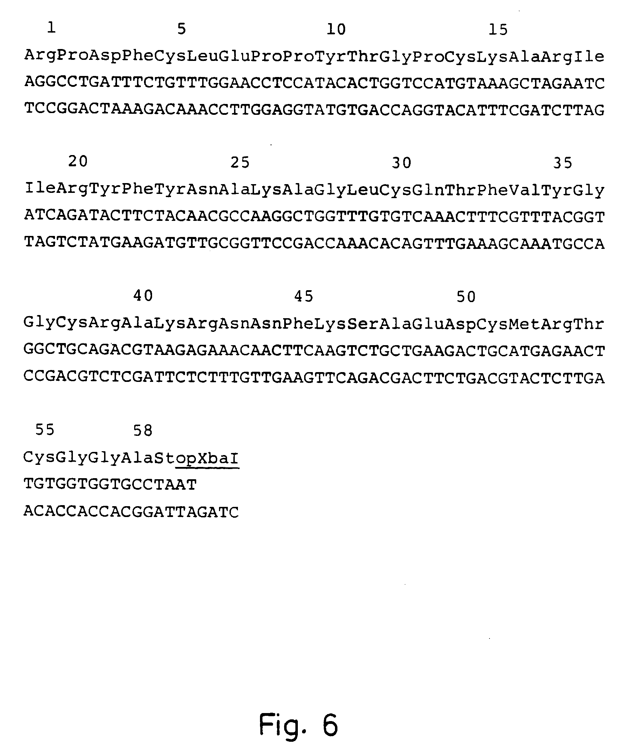

Fig. 6 shows a synthetic gene encoding aprotinin (1-58); and

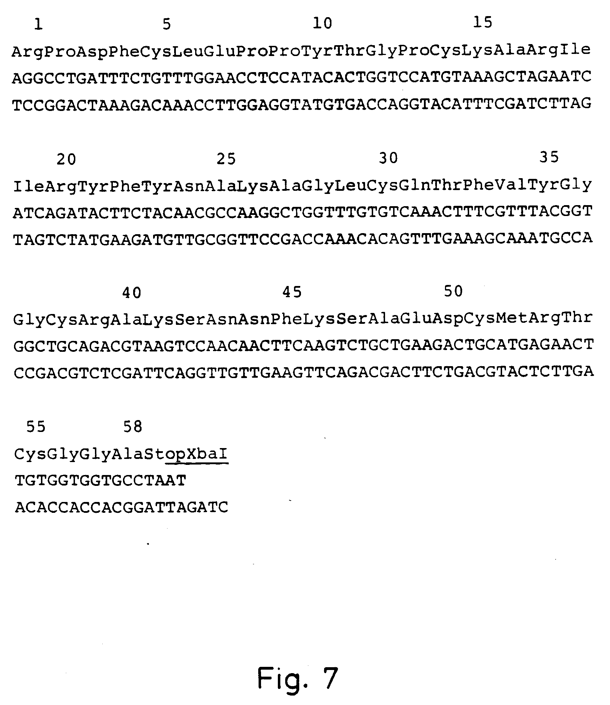

Fig. 7 shows a synthetic gene encoding aprotinin(1-58,42 Ser).

### DETAILED DESCRIPTION OF THE PRESENTLY PREFERRED EMBODIMENTS

For secretion purposes the DNA-sequence encoding the desired aprotinin or aprotinin homologue is fused to a DNA-sequence encoding a signal and leader peptide sequence. The signal and leader peptides are cleaved off by the transformed microorganism during the secretion of the expressed protein product from the cells ensuring a more simple isolation procedure of the desired product. A well suited leader peptide system for yeast is the yeast MFα1 leader sequence or a part thereof (Kurjan, J. and Herskowitz, I., Cell 30 (1982) 933-943) or a leader described in Danish patent application No. 4638/87. However, any signal- or leader-sequence which provides for secretion in yeast may be employed and the present invention is not contemplated to be restricted to a specific secretion system.

For expression purposes a promoter sequence is positioned upstream to the DNA-sequence for the desired protein product. Preferably a promoter from a gene indigenous to the yeast host organism is used, e.g. the promoter of the TPI-(triose phosphate isomerase) gene. The DNA-sequence for the desired product will be followed by a transcription terminator sequence, preferably a terminator sequence from a gene indigenous to the host yeast organism, e.g. the terminator of the TPI-gene or the MFα1 gene.

The DNA-sequence encoding the aprotinin or aprotinin homologue fused to appropriate promoter, signal, leader and terminator sequences is inserted into an expression vector for expression of the aprotinin or aprotinin homologue in yeast.

The expression vector may be a plasmid capable of independent replication in yeast or capable of integration into the yeast chromosome. The plasmid may preferably be stabilized against plasmid loss by the host microorganism by incorporation of a gene essential for the viability or normal growth of the host cells, e.g. a gene coding for cell division, cell wall biosynthesis, protein synthesis, etc.

As used in the present specification, aprotinin(1-58) means aprotinin having the amino acid sequence of native aprotinin whereas aprotinin(3-58) means an aprotinin homologue lacking the first two N-terminal amino acid residues. Aprotinin(1-58,42 Ser) means an aprotinin homologue in which Arg in position 42 has been replaced by Ser, and aprotinin (3-58,42 Ser) means an aprotinin homologue lacking the first two N-terminal amino acid residues and furthermore having Arg in position 42 replaced by Ser.

The methods for transformation of yeast and cultivation of transformed yeast strains that can be used in the practice of the invention are those known in the art such as those described in the above mentioned EP patent applications Nos. 0163529A and 0189998A.

To minimize possible complications during secretion caused by a "triple basic" cleavage site at the N-terminal end of the aprotinin gene an aprotinin homologue lacking the first two N-terminal amino acid residues (Arg-Pro) was produced.

Also, to avoid or minimize possible Kex2 cleavage of aprotinin at Lys(41)-Arg(42) an aprotinin homologue in which one or both of the amino acid residues Lys(41) and Arg(42) has been substituted with a non-basic amino acid residue can be prepared. A preferred aprotinin homologue of this type is one having Ser in position 42 instead of Arg.

Surprisingly, it was further demonstrated that irrespective of the fact that native aprotinin contains an N-terminal basic amino acid residue and contains a dibasic sequence, yeasts are still able to express and secrete a product identical with native aprotinin without modification of the aprotinin gene.

The present novel aprotinin homologues have the same specific inhibitory effect towards bovine trypsin as native aprotinin and may accordingly be used as a substitute for native aprotinin.

The specific inhibitory effect of the compounds of the invention was measured by following the inhibition of trypsin hydrolysis of benzoylarginine-p-nitroanilide (BAPNA) by spectrometric analysis as described by Erlanger,B.F., Kokonski, N., and Cohen,W. , Arch.Biochem.Biophys. 95, (1961) 271.

The DNA-sequences encoding the present aprotinin homologues are preferably made by oligonucleotide synthesis using known techniques because this makes it possible to select codons known to be preferred for yeast expression.

The following synthetic genes have been constructed:

AspPheCysLeuGluProProTyrThrGlyProCysLysAlaArgIleIleArg
GATTTCTGTTTGGAACCTCCATACACTGGTCCATGTAAAGCTAGAATCATCAGA
CTAAAGACAAACCTTGGAGGTATGTGACCAGGTACATTTCGATCTTAGTAGTCT

TyrPheTyrAsnAlaLysAlaGlyLeuCysGlnThrPheValTyrGlyGlyCys
TACTTCTACAACGCCAAGGCTGGTTTGTGTCAAACTTTCGTTTACGGTGGCTGC
ATGAAGATGTTGCGGTTCCGACCAAACACAGTTTGAAAGCAAATGCCACCGACG

ArgAlaLysArgAsnAsnPheLysSerAlaGluAspCysMetArgThrCysGly
AGAGCTAAGAGAAACAACTTCAAGTCTGCTGAAGACTGCATGAGAACTTGTGGT
TCTCGATTCTCTTTGTTGAAGTTCAGACGACTTCTGACGTACTCTTGAACACCA

GlyAla
GGTGCC
CCACGG      encoding aprotinin(3-58),

AspPheCysLeuGluProProTyrThrGlyProCysLysAlaArgIleIleArg
GATTTCTGTTTGGAACCTCCATACACTGGTCCATGTAAAGCTAGAATCATCAGA
CTAAAGACAAACCTTGGAGGTATGTGACCAGGTACATTTCGATCTTAGTAGTCT

TyrPheTyrAsnAlaLysAlaGlyLeuCysGlnThrPheValTyrGlyGlyCys
TACTTCTACAACGCCAAGGCTGGTTTGTGTCAAACTTTCGTTTACGGTGGCTGC
ATGAAGATGTTGCGGTTCCGACCAAACACAGTTTGAAAGCAAATGCCACCGACG

ArgAlaLysSerAsnAsnPheLysSerAlaGluAspCysMetArgThrCysGly
AGAGCTAAGTCCAACAACTTCAAGTCTGCTGAAGACTGCATGAGAACTTGTGGT
TCTCGATTCAGGTTGTTGAAGTTCAGACGACTTCTGACGTACTCTTGAACACCA

GlyAla
GGTGCC
CCACGG      encoding aprotinin(3-58,42 Ser),


AGGCCTGATTTCTGTTTGGAACCTCCATACACTGGTCCATGTAAAGCTAGAATC
TCCGGACTAAAGACAAACCTTGGAGGTATGTGACCAGGTACATTTCGATCTTAG


ATCAGATACTTCTACAACGCCAAGGCTGGTTTGTGTCAAACTTTCGTTTACGGT
TAGTCTATGAAGATGTTGCGGTTCCGACCAAACACAGTTTGAAAGCAAATGCCA


GGCTGCAGAGCTAAGAGAAACAACTTCAAGTCTGCTGAAGACTGCATGAGAACT
CCGACGTCTCGATTCTCTTTGTTGAAGTTCAGACGACTTCTGACGTACTCTTGA


TGTGGTGGTGCC
ACACCACCACGG     encoding aprotinin(1-58), and

ArgProAspPheCysLeuGluProProTyrThrGlyProCysLysAlaArgIle
AGGCCTGATTTCTGTTTGGAACCTCCATACACTGGTCCATGTAAAGCTAGAATC
TCCGGACTAAAGACAAACCTTGGAGGTATGTGACCAGGTACATTTCGATCTTAG

IleArgTyrPheTyrAsnAlaLysAlaGlyLeuCysGlnThrPheValTyrGly
ATCAGATACTTCTACAACGCCAAGGCTGGTTTGTGTCAAACTTTCGTTTACGGT
TAGTCTATGAAGATGTTGCGGTTCCGACCAAACACAGTTTGAAAGCAAATGCCA

GlyCysArgAlaLysSerAsnAsnPheLysSerAlaGluAspCysMetArgThr
GGCTGCAGAGCTAAGTCCAACAACTTCAAGTCTGCTGAAGACTGCATGAGAACT
CCGACGTCTCGATTCAGGTTGTTGAAGTTCAGACGACTTCTGACGTACTCTTGA

CysGlyGlyAla
TGTGGTGGTGCC
ACACCACCACGG     encoding aprotinin(1-58,42 Ser).

### DETAILED DESCRIPTION

### Example 1

### Production of aprotinin(3-58)

The synthetic gene for aprotinin(3-58) was constructed from a number of oligonucleotides by ligation.

The oligonucleotides were synthesized on an automatic DNA synthesizer using phosphoramidite chemistry on a controlled pore glass support (Beaucage, S.L., and Caruthers, M.H., Tetrahedron Letters 22 (1981) 1859-1869).

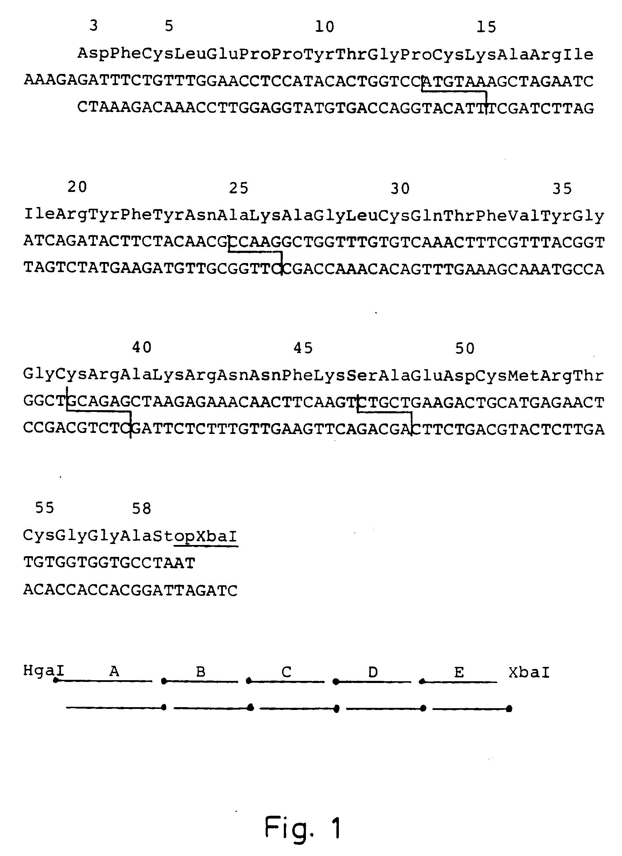

The following 10 oligonucleotides were synthesized: 
I:     AAAGAGATTTCTGTTTGGAACCTCCATACACTGGTCC
                    37-mer

II:    TTACATGGACCAGTGTATGGAGGTTCCAAACAGAAACT
                    38-mer

III:   ATGTAAAGCTAGAATCATCAGATACTTCTACAACG
                    35-mer

IV:    CTTGGCGTTGTAGAAGTATCTGATGATTCTAGCT
                    34-mer

V:     CCAAGGCTGGTTTGTGTCAAACTTTCGTTTACGGTGGCT
                    39-mer
 
VI:   CTCTGCAGCCACCGTAAACGAAAGTTTGACACAAACCAGC
40-mer

VII:   GCAGAGCTAAGAGAAACAACTTCAAGT
27-mer

VIII: AGCAGACTTGAAGTTGTTTCTCTTAG
26-mer

IX:   CTGCTGAAGACTGCATGAGAACTTGTGGTGGTGCCTAAT
39-mer

X:   CTAGATTAGGCACCACCACAAGTTCTCATGCAGTCTTC
38-mer
5 duplexes A - E were formed from the above 10 oligonucleotides as shown in fig. 1.

20 pmole of each of the duplexes A - E was formed from the corresponding pairs of 5'-phosphorylated oligonucleotides I - X by heating for 5 min. at 90°C followed by cooling to room temperature over a period of 75 minutes. The five duplexes were mixed and treated with T4 ligase. The synthetic gene was isolated as a 176 bp band after electrophoresis of the ligation mixture on a 2% agarose gel. The obtained synthetic gene is shown in fig. 1.

The synthetic gene was ligated to a 330 bp EcoRI-HgaI fragment from plasmid pKFN9 coding for MFα1 signal and leader sequence(1-85) and to the large EcoRI-XbaI fragment from pUC19. The construction of pKFN9 containing a HgaI site immediately after the MFα1 leader sequence is described in EP-patent application No. 0214826.

The ligation mixture was used to transform a competent E. coli strain (r⁻, m⁺) selecting for ampicillin resistance.Sequencing of a ³²P-XbaI-EcoRI fragment (Maxam, A and Gilbert, W., Methods Enzymol. 65 (1980) 499-560) showed that plasmids from the resulting colonies contained the correct DNA-sequence for aprotinin(3-58).

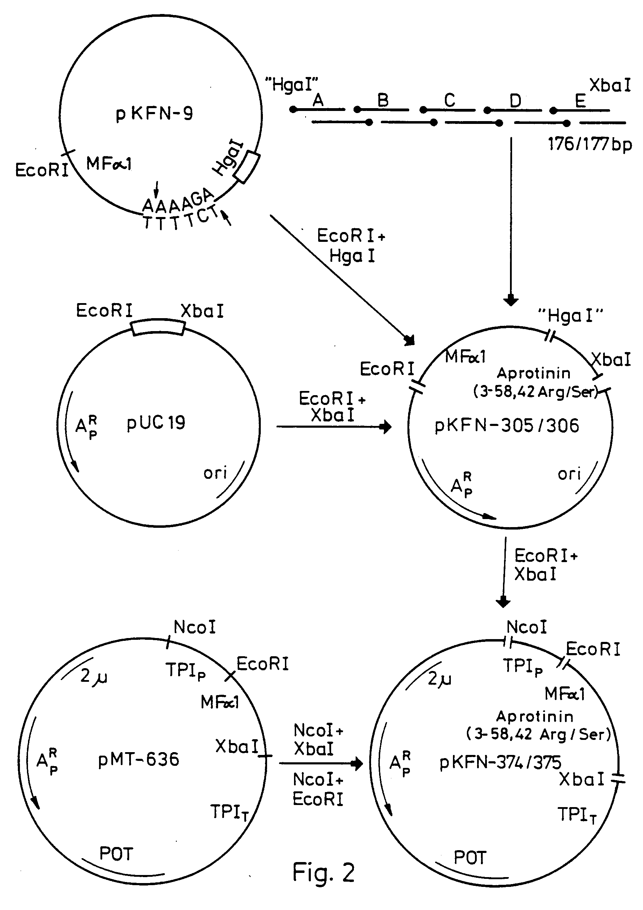

One plasmid pKNF305 was selected for further use. The construction of plasmid pKFN305 is illustrated in fig. 2.

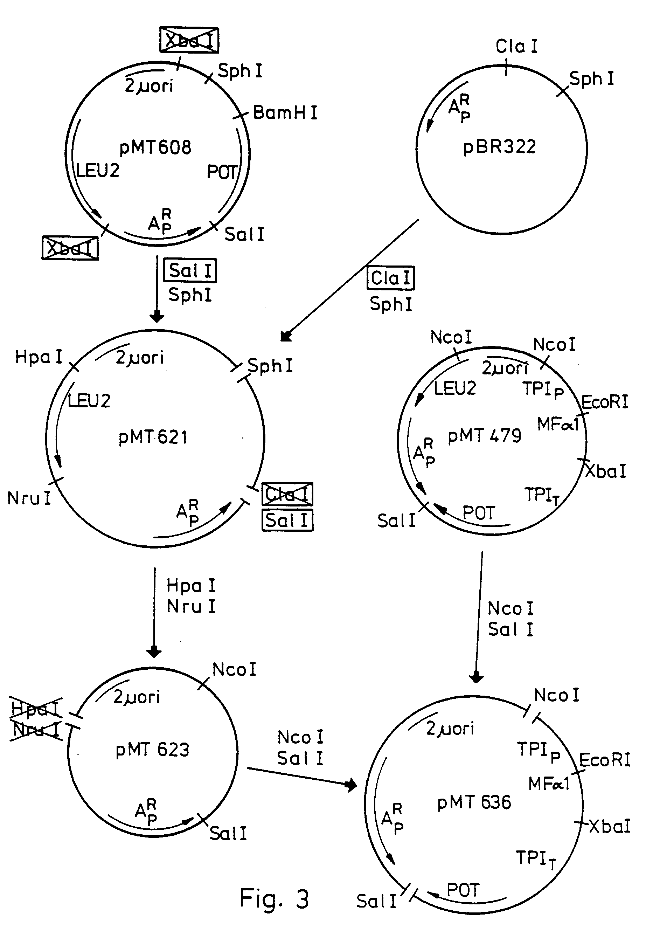

pKFN305 was cut with EcoRI and XbaI and the 0.5 kb fragment was ligated to the 9.5 kb NcoI-XbaI fragment from pMT636 and the 1.4 kb NcoI-EcoRI fragment from pMT636, resulting in plasmid pKFN374, see fig. 2. Plasmid pMT636 was constructed from pMT608 after deletion of the LEU-2 gene and from pMT479, see fig. 3. pMT608 is described in EP-patent application No. 195691. pMT479 is described in EP-patent application No. 163529. pMT479 contains the Schizo. pombe TPI gene (POT), the S. cerevisiae triosephosphate isomerase promoter and terminator, TPI_{P} and TPI_{T} (Alber, T. and Kawasaki, G. J.Mol.Appl.Gen. 1 (1982) 419-434). Plasmid pKFN374 contains the following sequence:
TPI_{P}-MFα1-signal-leader(1-85)-aprotinin(3-58)-TPI_{T}.
where MFα1 is the S. cerevisiae mating factor alpha 1 coding sequence (Kurjan, J. and Herskowitz, I., Cell 30, (1982) 933-943), signal-leader(1-85) means that the sequence contains the first 85 amino acid residues of the MFα1 signal-leader sequence and aprotinin(3-58) is the synthetic sequence encoding an aprotinin derivative lacking the first two amino acid residues.

S. cerevisiae strain MT663 (E2-7B XE11-36 a/α, ΔtpiΔtpi, pep 4-3/pep 4-3) was grown on YPGaL (1% Bacto yeast extract, 2% Bacto peptone, 2% galactose, 1% lactate) to an O.D. at 600 nm of 0.6.

100 ml of culture was harvested by centrifugation, washed with 10 ml of water, recentrifuged and resuspended in 10 ml of a solution containing 1.2 M sorbitol, 25 mM Na₂EDTA pH = 8.0, and 6.7 mg/ml dithiotreitol. The suspension was incubated at 30°C for 15 minutes, centrifuged and the cells resuspended in 10 ml of a solution containing 1.2 M sorbitol, 10 mM Na₂EDTA, 0.1 M sodium citrate, pH = 5.8, and 2 mg Novozym® 234. The suspension was incubated at 30°C for 30 minutes, the cells collected by centrifugation, washed in 10 ml of 1.2 M sorbitol and 10 ml of CAS (1.2 M sorbitol, 10 mM CaCl₂, 10 mM Tris HCl (Tris = Tris(hydroxymethyl)aminomethan) pH = 7.5) and resuspended in 2 ml of CAS. For transformation 0.1 ml of CAS-resuspended cells were mixed with approximately 1 µg of plasmid pKFN374 and left at room temperature for 15 minutes. 1 ml of (20% polyethylenglycol 4000, 10 mM CaCl₂, 10 mM Tris HCl, pH = 7.5) was added and the mixture left for further 30 minutes at room temperature. The mixture was centrifuged and the pellet resuspended in 0.1 ml of SOS (1.2 M sorbitol, 33% v/v YPD, 6.7 mM CaCl₂, 14 µg/ml leucine) and incubated at 30°C for 2 hours. The suspension was then centrifuged and the pellet resuspended in 0.5 ml of 1.2 M sorbitol. Then, 6 ml of top agar (the SC medium of Sherman et al., (Methods in Yeast Genetics, Cold Spring Harbor Laboratory, 1981) containing 1.2 M sorbitol plus 2.5% agar) at 52°C was added and the suspension poured on top of plates containing the same agar-solidified, sorbitol containing medium. Transformant colonies were picked after 3 days at 30°C, reisolated and used to start liquid cultures. One such transformant KFN322 was chosen for further characterization.

Yeast strain KFN322 was grown on YPD medium (1% yeast extract, 2% peptone (from Difco Laboratories), and 2% glucose). A 10 ml culture of the strain was shaken at 30°C to an O.D. at 600 nm of 32. After centrifugation the supernatant was analyzed by FPLC ion exchange chromatography. The yeast supernatant was filtered through a 0.22 µm Millex® GV filter unit and 1 ml was applied on a MonoS cation exchange column (0.5 x 5 cm) equilibrated with 20 mM Bicine, pH 8.7. After wash with equilibration buffer the column was eluted with a linear NaCl gradient (0-1 M) in equilibration buffer. Trypsin inhibitor activity was quantified in the eluted fractions by spectrophotometric assay and furthermore by integration of absorption at 280 nm from 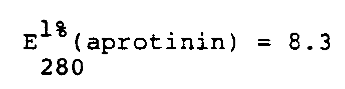
The yield was about 3 mg/liter of aprotinin(3-58).

For amino acid analysis and N-terminal sequencing the yeast supernatant (7 ml) was adjusted to pH 8.7 with 0.1 M NaOH and filtered (0.22 µm). The effluent from a Q-Sepharose anion exchange column (1 x 4 cm) equilibrated with 20 mM Bicine, pH 8.7 was applied to a MonoS cation exchange column (0.5 x 5 cm). The cation exchange chromatography was carried out as described above. Concentration of the gradient eluated aprotinin(3-58) was accomplished by rechromatography on MonoS and elution with a steep NaCl-gradient. The collected fractions were further concentrated by vacuum centrifugation to about 100 µl and applied to a RP-HPLC column (Vydac C4, 4.6 x 250 mm). Elution was carried out with CH₃CN gradient in 0.1% TFA. The collected fractions were concentrated to about 100 µl by vacuum centrifugation and samples were taken for N-terminal sequencing and amino acid analysis.

By N-terminal sequencing the following sequence was found. 
Asp-Phe-Cys-Leu-Glu-Pro-Pro-Tyr-Thr-Gly-Pro-Cys-Lys-
Ala-Arg-Ile-Ile-Arg
confirming that the N-terminal end is correct.

The amino acid analysis is shown in following Table 1. From this table it appears that the product has the expected amino acid composition, i.e. less Arg and Pro. The slightly lowered content of Ile can most probably be ascribed incomplete hydrolysis of Ile(18)-Ile(19) (this is well known in the art). Also, Pro and Arg is slightly higher than expected. This is, however, also seen with aprotinin itself (Table 1, second column).

When compared by the above-mentioned method of Erlanger et al. the specific activity of aprotinin(3-58) was found to be identical within the experimental error with the specific activity of native aprotinin.

### Example 2

### Production of aprotinin(3-58,42 Ser).

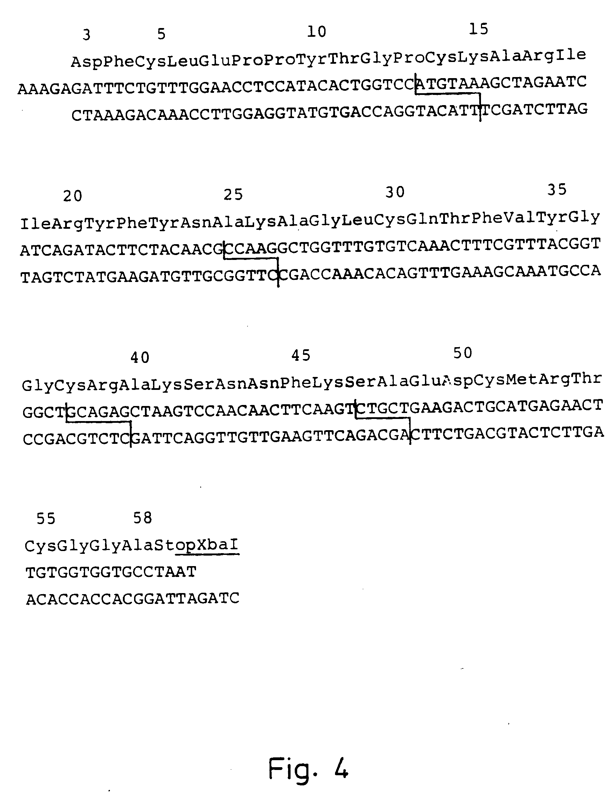

A synthetic gene for aprotinin(3-58,42 Ser) was constructed as described in example 1. With the purpose of substituting Arg(42) with Ser the following oligonucleotides VIIa and VIIIa were used instead of VII and VIII: 
VIIa:    GCAGAGCTAAGTCCAACAACTTCAAGT
27-mer

VIIIa:    AGCAGACTTGAAGTTGTTGGACTTAG
26-mer
The obtained synthetic gene is shown in fig. 4. This gene fused to the MFα1 signal-leader(1-85) sequence was cloned in a pUC19 derived plasmid pKFN306 (see fig. 2).

By following the procedure of example 1 a plasmid pKFN375 was obtained containing the following construction
TPI_{P}-MFα1-signal-leader(1-85)-aprotinin(3-58,42 Ser)-TPI_{T}
where aprotinin(3-58,42 Ser) is the synthetic gene encoding an aprotinin derivative lacking the first two amino acid residues and containing a Ser instead of Arg in position 42.

Yeast strain MT663 was transformed with plasmid pKFN375 as described above, and culturing of the transformed strain KFN324 gave about 12 mg/liter of aprotinin(3-58,42 Ser).

N-terminal sequencing carried out as described above confirmed the following N-terminal sequence 
Asp-Phe-Cys-Leu-Glu-Pro-Pro-Tyr-Thr-Gly-Pro-Cys-Lys-
Ala-Arg-Ile-Ile-Arg-Tyr-Phe
i.e. the correct sequence.

The amino acid analysis is shown in Table 1 and confirms the expected amino acid composition, i.e. less Pro and Arg and more Ser (see also the above remarks in Example 1).

When compared by the above-mentioned method of Erlanger et al. the specific activity of aprotinin(3-58,42 Ser) was found to be identical within the experimental error with the specific activity of native aprotinin.

### Example 3

### Production of aprotinin(1-58)

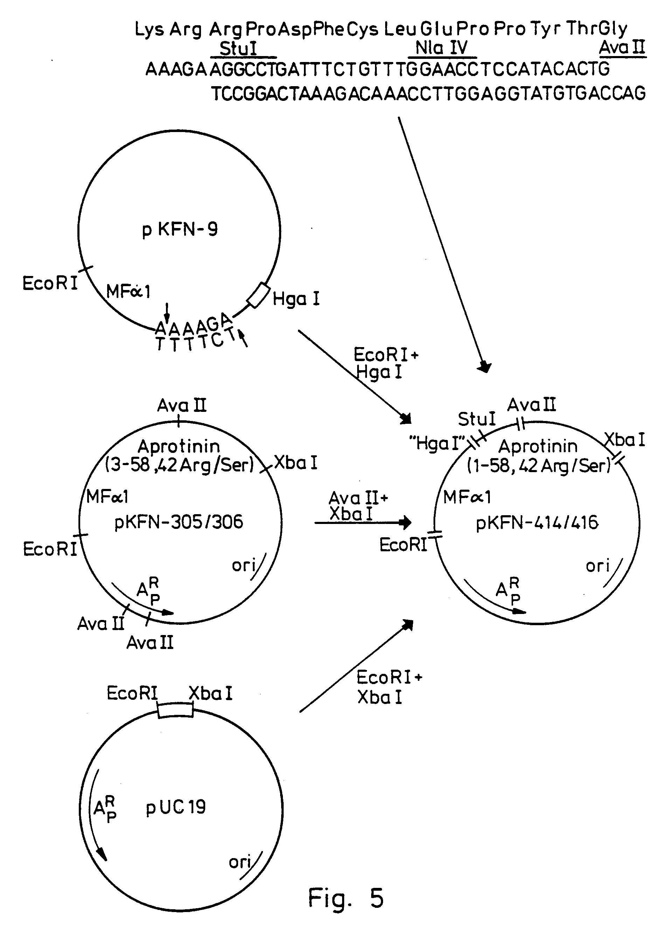

The synthetic duplex shown in fig. 5 was ligated to the 330 bp EcoRI-HgaI fragment from plasmid pKFN9 coding for MFα1 signal and leader sequence and to the 144 bp AvaII-XbaI fragment from pKFN305 and to the large EcoRI-XbaI fragment from pUC19.

The ligation mixture was used to transform a competent E. coli strain (r⁻ m⁺) selecting for ampicillin resistance. Sequencing of a ³²P-labelled XbaI-EcoRI fragment showed that plasmids from the resulting colonies contained the correct DNA sequence for aprotinin(1-58).

One plasmid pKFN414 was selected for further use. The construction of plasmid pKFN414 is illustrated in fig. 5.

By following the procedure of example 1 a yeast plasmid pKFN418 was obtained containing the following construction:
TPI_{P}-MFα1-signal-leader(1-85)-aprotinin(1-58)-TPI_{T}
Yeast strain MT633 was transformed with plasmid pKFN418 as described above. Culturing of the transformed strain KFN385 gave about 1-13 mg/l of aprotinin(1-58).

When compared by the above-mentioned method of Erlanger et al. the specific activity of aprotinin(1-58), produced according to this example was found to be identical within the experimental error with the specific activity of native aprotinin.

### Example 4

### Production of aprotinin(1-58,42 Ser)

A plasmid pKFN416 containing a gene for aprotinin(1-58,42 Ser) was constructed from pKFN306 as described in example 3. By following the procedure of example 1 a yeast plasmid pKFN420 was obtained containing the following construction:
TPI_{P}-MFα1-signal-leader(1-85)-aprotinin(1-58,42 Ser)-TPI_{T}
Yeast strain MT663 was transformed with plasmid pKFN420 as described above. Culturing of the transformed strain KFN387 gave about 1-13 mg/l of aprotinin(1-58,42 Ser).

When compared by the above-mentioned method of Erlanger et al. the specific activity of aprotinin(1-58,42 Ser) was found to be identical within the experimental error with the specific activity of native aprotinin.

**Table 1**

| Amino acid | Aprotinin (theoretical) | Aprotinin (found) | Aprotinin (3-58) (found) | Aprotinin (3-58,42 Ser) (found) |
|---|---|---|---|---|
| Asx | 5 | 5.00 | 4,96 | 5.02 |
| Thr | 3 | 2.86 | 2.83 | 2.85 |
| Ser | 1 | 0.94 | 0.97 | 1.78 |
| Glx | 3 | 3.04 | 3 01 | 3.02 |
| Pro | 4 | 4.18 | 3.15 | 3.19 |
| Gly | 6 | 5.95 | 6.00 | 5.99 |
| Ala | 6 | 5.85 | 5.93 | 6.01 |
| Cys | 6 | 5.20 | 5.03 | 5.41 |
| Val | 1 | 0.99 | 0.98 | 0.98 |
| Met | 1 | 0.83 | 0.85 | 0.96 |
| Ile | 2 | 1.39 | 1.41 | 1.50 |
| Leu | 2 | 1.97 | 1.98 | 2.03 |
| Tyr | 4 | 3.84 | 3.80 | 3.82 |
| Phe | 4 | 3.98 | 3.92 | 3.96 |
| Lys | 4 | 3.92 | 4.02 | 3.93 |
| Arg | 6 | 6.39 | 5.20 | 4.28 |
| Total | 58 | 56.33 | 54.04 | 54.73 |

## Claims

1. A process for producing aprotinin or homologues thereof in yeast comprising culturing a yeast strain containing a replicable expression vector, the vector comprising a DNA sequence encoding aprotinin or an aprotinin homologue fused to a DNA sequence encoding a signal and leader peptide providing for secretion of the aprotinin or aprotinin homologue, in a suitable nutrient medium and recovering the secreted aprotinin or aprotinin homologue therefrom.

2. A process according to claim 1, wherein the DNA sequence encodes aprotinin(3-58).

3. A process according to claim 2, wherein the DNA sequence has the following sequence: 
AspPheCysLeuGluProProTyrThrGlyProCysLysAlaArgIleIleArg
GATTTCTGTTTGGAACCTCCATACACTGGTCCATGTAAAGCTAGAATCATCAGA
CTAAAGACAAACCTTGGAGGTATGTGACCAGGTACATTTCGATCTTAGTAGTCT

TyrPheTyrAsnAlaLysAlaGlyLeuCysGlnThrPheValTyrGlyGlyCys
TACTTCTACAACGCCAAGGCTGGTTTGTGTCAAACTTTCGTTTACGGTGGCTGC
ATGAAGATGTTGCGGTTCCGACCAAACACAGTTTGAAAGCAAATGCCACCGACG

ArgAlaLysArgAsnAsnPheLysSerAlaGluAspCysMetArgThrCysGly
AGAGCTAAGAGAAACAACTTCAAGTCTGCTGAAGACTGCATGAGAACTTGTGGT
TCTCGATTCTCTTTGTTGAAGTTCAGACGACTTCTGACGTACTCTTGAACACCA

GlyAla
GGTGCC
CCACGG
 or a functionally equivalent sequence.

4. A process according to claim 1, wherein the DNA sequence encodes aprotinin(3-58,42 Ser).

5. A process according to claim 4, wherein the DNA sequence has the following sequence 
AspPheCysLeuGluProProTyrThrGlyProCysLysAlaArgIleIleArg
GATTTCTGTTTGGAACCTCCATACACTGGTCCATGTAAAGCTAGAATCATCAGA
CTAAAGACAAACCTTGGAGGTATGTGACCAGGTACATTTCGATCTTAGTAGTCT

TyrPheTyrAsnAlaLysAlaGlyLeuCysGlnThrPheValTyrGlyGlyCys
TACTTCTACAACGCCAAGGCTGGTTTGTGTCAAACTTTCGTTTACGGTGGCTGC
ATGAAGATGTTGCGGTTCCGACCAAACACAGTTTGAAAGCAAATGCCACCGACG

ArgAlaLysSerAsnAsnPheLysSerAlaGluAspCysMetArgThrCysGly
AGAGCTAAGTCCAACAACTTCAAGTCTGCTGAAGACTGCATGAGAACTTGTGGT
TCTCGATTCAGGTTGTTGAAGTTCAGACGACTTCTGACGTACTCTTGAACACCA

GlyAla
GGTGCC
CCACGG
 or a functionally equivalent sequence.

6. A process according to claim 1, wherein the DNA sequence encodes aprotinin(1-58).

7. A process according to claim 6, wherein the DNA sequence has the following sequence 

AGGCCTGATTTCTGTTTGGAACCTCCATACACTGGTCCATGTAAAGCTAGAATC
TCCGGACTAAAGACAAACCTTGGAGGTATGTGACCAGGTACATTTCGATCTTAG


ATCAGATACTTCTACAACGCCAAGGCTGGTTTGTGTCAAACTTTCGTTTACGGT
TAGTCTATGAAGATGTTGCGGTTCCGACCAAACACAGTTTGAAAGCAAATGCCA


GGCTGCAGAGCTAAGAGAAACAACTTCAAGTCTGCTGAAGACTGCATGAGAACT
CCGACGTCTCGATTCTCTTTGTTGAAGTTCAGACGACTTCTGACGTACTCTTGA
 

TGTGGTGGTGCC
ACACCACCACGG
 or a functionally equivalent sequence.

8. A process according to claim 1, wherein the DNA sequence encodes aprotinin(1-58,42 Ser).

9. A process according to claim 8, wherein the DNA sequence has the following nucleotide sequence 
ArgProAspPheCysLeuGluProProTyrThrGlyProCysLysAlaArgIle
AGGCCTGATTTCTGTTTGGAACCTCCATACACTGGTCCATGTAAAGCTAGAATC
TCCGGACTAAAGACAAACCTTGGAGGTATGTGACCAGGTACATTTCGATCTTAG

IleArgTyrPheTyrAsnAlaLysAlaGlyLeuCysGlnThrPheValTyrGly
ATCAGATACTTCTACAACGCCAAGGCTGGTTTGTGTCAAACTTTCGTTTACGGT
TAGTCTATGAAGATGTTGCGGTTCCGACCAAACACAGTTTGAAAGCAAATGCCA

GlyCysArgAlaLysSerAsnAsnPheLysSerAlaGluAspCysMetArgThr
GGCTGCAGAGCTAAGTCCAACAACTTCAAGTCTGCTGAAGACTGCATGAGAACT
CCGACGTCTCGATTCAGGTTGTTGAAGTTCAGACGACTTCTGACGTACTCTTGA

CysGlyGlyAla
TGTGGTGGTGCC
ACACCACCACGG
 or a functionally equivalent sequence.

10. A vector capable of replicating in yeast and comprising a DNA sequence encoding aprotinin or an aprotinin homologue fused to a DNA-sequence encoding a signal and leader peptide providing for secretion of the aprotinin or aprotinin homologue.

11. A vector according to claim 10, wherein the DNA sequence codes for aprotinin(3-58) and has the following sequence 
AspPheCysLeuGluProProTyrThrGlyProCysLysAlaArgIleIleArg
GATTTCTGTTTGGAACCTCCATACACTGGTCCATGTAAAGCTAGAATCATCAGA
CTAAAGACAAACCTTGGAGGTATGTGACCAGGTACATTTCGATCTTAGTAGTCT
 
TyrPheTyrAsnAlaLysAlaGlyLeuCysGlnThrPheValTyrGlyGlyCys
TACTTCTACAACGCCAAGGCTGGTTTGTGTCAAACTTTCGTTTACGGTGGCTGC
ATGAAGATGTTGCGGTTCCGACCAAACACAGTTTGAAAGCAAATGCCACCGACG

ArgAlaLysArgAsnAsnPheLysSerAlaGluAspCysMetArgThrCysGly
AGAGCTAAGAGAAACAACTTCAAGTCTGCTGAAGACTGCATGAGAACTTGTGGT
TCTCGATTCTCTTTGTTGAAGTTCAGACGACTTCTGACGTACTCTTGAACACCA

GlyAla
GGTGCC
CCACGG
 or a functionally equivalent sequence.

12. A vector according to claim 10, wherein the DNA sequence codes for aprotinin(3-58,42 Ser) and has the following sequence 
AspPheCysLeuGluProProTyrThrGlyProCysLysAlaArgIleIleArg
GATTTCTGTTTGGAACCTCCATACACTGGTCCATGTAAAGCTAGAATCATCAGA
CTAAAGACAAACCTTGGAGGTATGTGACCAGGTACATTTCGATCTTAGTAGTCT

TyrPheTyrAsnAlaLysAlaGlyLeuCysGlnThrPheValTyrGlyGlyCys
TACTTCTACAACGCCAAGGCTGGTTTGTGTCAAACTTTCGTTTACGGTGGCTGC
ATGAAGATGTTGCGGTTCCGACCAAACACAGTTTGAAAGCAAATGCCACCGACG

ArgAlaLysSerAsnAsnPheLysSerAlaGluAspCysMetArgThrCysGly
AGAGCTAAGTCCAACAACTTCAAGTCTGCTGAAGACTGCATGAGAACTTGTGGT
TCTCGATTCAGGTTGTTGAAGTTCAGACGACTTCTGACGTACTCTTGAACACCA

GlyAla
GGTGCC
CCACGG
 or a functionally equivalent sequence.

13. A vector according to claim 10 wherein the DNA sequence codes for aprotinin(1-58) and has the following sequence 

AGGCCTGATTTCTGTTTGGAACCTCCATACACTGGTCCATGTAAAGCTAGAATC
TCCGGACTAAAGACAAACCTTGGAGGTATGTGACCAGGTACATTTCGATCTTAG
 

ATCAGATACTTCTACAACGCCAAGGCTGGTTTGTGTCAAACTTTCGTTTACGGT
TAGTCTATGAAGATGTTGCGGTTCCGACCAAACACAGTTTGAAAGCAAATGCCA


GGCTGCAGAGCTAAGAGAAACAACTTCAAGTCTGCTGAAGACTGCATGAGAACT
CCGACGTCTCGATTCTCTTTGTTGAAGTTCAGACGACTTCTGACGTACTCTTGA


TGTGGTGGTGCC
ACACCACCACGG
 or a functionally equivalent sequence.

14. A vector according to claim 10, wherein the DNA sequence codes for aprotinin(1-58,42 Ser) and has the following sequence 
ArgProAspPheCysLeuGluProProTyrThrGlyProCysLysAlaArgIle
AGGCCTGATTTCTGTTTGGAACCTCCATACACTGGTCCATGTAAAGCTAGAATC
TCCGGACTAAAGACAAACCTTGGAGGTATGTGACCAGGTACATTTCGATCTTAG

IleArgTyrPheTyrAsnAlaLysAlaGlyLeuCysGlnThrPheValTyrGly
ATCAGATACTTCTACAACGCCAAGGCTGGTTTGTGTCAAACTTTCGTTTACGGT
TAGTCTATGAAGATGTTGCGGTTCCGACCAAACACAGTTTGAAAGCAAATGCCA

GlyCysArgAlaLysSerAsnAsnPheLysSerAlaGluAspCysMetArgThr
GGCTGCAGAGCTAAGTCCAACAACTTCAAGTCTGCTGAAGACTGCATGAGAACT
CCGACGTCTCGATTCAGGTTGTTGAAGTTCAGACGACTTCTGACGTACTCTTGA

CysGlyGlyAla
TGTGGTGGTGCC
ACACCACCACGG
 or a functionally equivalent sequence.

15. A yeast strain transformed with a vector according to claim 10.

## Patentansprüche

1. Ein Verfahren zur Herstellung von Aprotinin oder Homologen desselben in Hefe, welches das Kultivieren eines Hefestammes, der einen replizierbaren Expressionsvektor enthält, wobei der Vektor eine DNA-Sequenz umfaßt, die Aprotinin oder ein Aprotinin-Homolog kodiert, gebunden an eine DNA-Sequenz, die ein Signal- und Leaderpeptid kodiert, das für Sekretion des Aprotinins oder Aprotinin-Homologs sorgt, in einem geeigneten Nährstoffmedium und die Gewinnung des sekretierten Aprotinins oder Aprotinin-Homologs daraus umfaßt.

2. Ein Verfahren nach Anspruch 1, wobei die DNA-Sequenz Aprotinin(3-58) kodiert.

3. Ein Verfahren nach Anspruch 2, wobei die DNA-Sequenz die folgende Sequenz besitzt: 
AspPheCysLeuGluProProTyrThrGlyProCysLysAlaArgIleIleArg
GATTTCTGTTTGGAACCTCCATACACTGGTCCATGTAAAGCTAGAATCATCAGA
CTAAAGACAAACCTTGGAGGTATGTGACCAGGTACATTTCGATCTTAGTAGTCT

TyrPheTyrAsnAlaLysAlaGlyLeuCysGlnThrPheValTyrGlyGlyCys
TACTTCTACAACGCCAAGGCTGGTTTGTGTCAAACTTTCGTTTACGGTGGCTGC
ATGAAGATGTTGCGGTTCCGACCAAACACAGTTTGAAAGCAAATGCCACCGACG

ArgAlaLysArgAsnAsnPheLysSerAlaGluAspCysMetArgThrCysGly
AGAGCTAAGAGAAACAACTTCAAGTCTGCTGAAGACTGCATGAGAACTTGTGGT
TCTCGATTCTCTTTGTTGAAGTTCAGACGACTTCTGACGTACTCTTGAACACCA

GlyAla
GGTGCC
CCACGG
 oder eine funktionell äquivalente Sequenz.

4. Ein Verfahren nach Anspruch 1, wobei die DNA-Sequenz Aprotinin(3-58,42 Ser) kodiert.

5. Ein Verfahren nach Anspruch 4, wobei die DNA-Sequenz die folgende Sequenz besitzt: 
AspPheCysLeuGluProProTyrThrGlyProCysLysAlaArgIleIleArg
GATTTCTGTTTGGAACCTCCATACACTGGTCCATGTAAAGCTAGAATCATCAGA
CTAAAGACAAACCTTGGAGGTATGTGACCAGGTACATTTCGATCTTAGTAGTCT

TyrPheTyrAsnAlaLysAlaGlyLeuCysGlnThrPheValTyrGlyGlyCys
TACTTCTACAACGCCAAGGCTGGTTTGTGTCAAACTTTCGTTTACGGTGGCTGC
ATGAAGATGTTGCGGTTCCGACCAAACACAGTTTGAAAGCAAATGCCACCGACG

ArgAlaLysSerAsnAsnPheLysSerAlaGluAspCysMetArgThrCysGly
AGAGCTAAGTCCAACAACTTCAAGTCTGCTGAAGACTGCATGAGAACTTGTGGT
TCTCGATTCAGGTTGTTGAAGTTCAGACGACTTCTGACGTACTCTTGAACACCA

GlyAla
GGTGCC
CCACGG
 oder eine funktionell äquivalente Sequenz.

6. Ein Verfahren nach Anspruch 1, wobei die DNA-Sequenz Aprotinin(1-58) kodiert.

7. Ein Verfahren nach Anspruch 6, wobei die DNA-Sequenz die folgende Sequenz besitzt: 

AGGCCTGATTTCTGTTTGGAACCTCCATACACTGGTCCATGTAAAGCTAGAATC
TCCGGACTAAAGACAAACCTTGGAGGTATGTGACCAGGTACATTTCGATCTTAG


ATCAGATACTTCTACAACGCCAAGGCTGGTTTGTGTCAAACTTTCGTTTACGGT
TAGTCTATGAAGATGTTGCGGTTCCGACCAAACACAGTTTGAAAGCAAATGCCA


GGCTGCAGAGCTAAGAGAAACAACTTCAAGTCTGCTGAAGACTGCATGAGAACT
CCGACGTCTCGATTCTCTTTGTTGAAGTTCAGACGACTTCTGACGTACTCTTGA


TGTGGTGGTGCC
ACACCACCACGG ,
 oder eine funktionell äquivalente Sequenz.

8. Ein Verfahren nach Anspruch 1, wobei die DNA-Sequenz Aprotinin(1-58,42 Ser) kodiert.

9. Ein Verfahren nach Anspruch 8, wobei die DNA-Sequenz die folgende Nukleotidsequenz besitzt 
ArgProAspPheCysLeuGluProProTyrThrGlyProCysLysAlaArgIle
AGGCCTGATTTCTGTTTGGAACCTCCATACACTGGTCCATGTAAAGCTAGAATC
TCCGGACTAAAGACAAACCTTGGAGGTATGTGACCAGGTACATTTCGATCTTAG

IleArgTyrPheTyrAsnAlaLysAlaGlyLeuCysGlnThrPheValTyrGly
ATCAGATACTTCTACAACGCCAAGGCTGGTTTGTGTCAAACTTTCGTTTACGGT
TAGTCTATGAAGATGTTGCGGTTCCGACCAAACACAGTTTGAAAGCAAATGCCA

GlyCysArgAlaLysSerAsnAsnPheLysSerAlaGluAspCysMetArgThr
GGCTGCAGAGCTAAGTCCAACAACTTCAAGTCTGCTGAAGACTGCATGAGAACT
CCGACGTCTCGATTCAGGTTGTTGAAGTTCAGACGACTTCTGACGTACTCTTGA

CysGlyGlyAla
TGTGGTGGTGCC
ACACCACCACGG ,
 oder eine funktionell äquivalente Sequenz.

10. Ein Vektor, der zu Replikation in Hefe in der Lage ist und eine DNA-Sequenz umfaßt, die Aprotinin oder ein Aprotinin-Homolog kodiert, gebunden an eine DNA-Sequenz, die ein Signal- und Leaderpeptid kodiert, das für Sekretion des Aprotinins oder Aprotinin-Homologs sorgt.

11. Ein Vektor nach Anspruch 10, wobei die DNA-Sequenz für Aprotinin(3-58) kodiert und die folgende Sequenz besitzt 
AspPheCysLeuGluProProTyrThrGlyProCysLysAlaArgIleIleArg
GATTTCTGTTTGGAACCTCCATACACTGGTCCATGTAAAGCTAGAATCATCAGA
CTAAAGACAAACCTTGGAGGTATGTGACCAGGTACATTTCGATCTTAGTAGTCT

TyrPheTyrAsnAlaLysAlaGlyLeuCysGlnThrPheValTyrGly[?]lyCys
TACTTCTACAACGCCAAGGCTGGTTTGTGTCAAACTTTCGTTTACGGTGGCTGC
ATGAAGATGTTGCGGTTCCGACCAAACACAGTTTGAAAGCAAATGCCACCGACG

ArgAlaLysArgAsnAsnPheLysSerAlaGluAspCysMetArgThrCysGly
AGAGCTAAGAGAAACAACTTCAAGTCTGCTGAAGACTGCATGAGAACTTGTGGT
TCTCGATTCTCTTTGTTGAAGTTCAGACGACTTCTGACGTACTCTTGAACACCA

GlyAla
GGTGCC
CCACGG ,
 oder eine funktionell äquivalente Sequenz.

12. Ein Vektor nach Anspruch 10, wobei die DNA-Sequenz für Aprotinin(3-58,42 Ser) kodiert und die folgende Sequenz besitzt 
AspPheCysLeuGluProProTyrThrGlyProCysLysAlaArgIleIleArg
GATTTCTGTTTGGAACCTCCATACACTGGTCCATGTAAAGCTAGAATCATCAGA
CTAAAGACAAACCTTGGAGGTATGTGACCAGGTACATTTCGATCTTAGTAGTCT

TyrPheTyrAsnAlaLysAlaGlyLeuCysGlnThrPheValTyrGlyGlyCys
TACTTCTACAACGCCAAGGCTGGTTTGTGTCAAACTTTCGTTTACGGTGGCTGC
ATGAAGATGTTGCGGTTCCGACCAAACACAGTTTGAAAGCAAATGCCACCGACG

ArgAlaLysSerAsnAsnPheLysSerAlaGluAspCysMetArgThrCysGly
AGAGCTAAGTCCAACAACTTCAAGTCTGCTGAAGACTGCATGAGAACTTGTGGT
TCTCGATTCAGGTTGTTGAAGTTCAGACGACTTCTGACGTACTCTTGAACACCA
 
GlyAla
GGTGCC
CCACGG ,
 oder eine funktionell äquivalente Sequenz.

13. Ein Vektor nach Anspruch 10, wobei die DNA-Sequenz für Aprotinin(1-58) kodiert und die folgende Sequenz besitzt 

AGGCCTGATTTCTGTTTGGAACCTCCATACACTGGTCCATGTAAAGCTAGAATC
TCCGGACTAAAGACAAACCTTGGAGGTATGTGACCAGGTACATTTCGATCTTAG


ATCAGATACTTCTACAACGCCAAGGCTGGTTTGTGTCAAACTTTCGTTTACGGT
TAGTCTATGAAGATGTTGCGGTTCCGACCAAACACAGTTTGAAAGCAAATGCCA


GGCTGCAGAGCTAAGAGAAACAACTTCAAGTCTGCTGAAGACTGCATGAGAACT
CCGACGTCTCGATTCTCTTTGTTGAAGTTCAGACGACTTCTGACGTACTCTTGA


TGTGGTGGTGCC
ACACCACCACGG ,
 oder eine funktionell äquivalente Sequenz.

14. Ein Vektor nach Anspruch 10, wobei die DNA-Sequenz für Aprotinin(1-58,42 Ser) kodiert und die folgende Sequenz besitzt 
ArgProAspPheCysLeuGluProProTyrThrGlyProCysLysAlaArgIle
AGGCCTGATTTCTGTTTGGAACCTCCATACACTGGTCCATGTAAAGCTAGAATC
TCCGGACTAAAGACAAACCTTGGAGGTATGTGACCAGGTACATTTCGATCTTAG

IleArgTyrPheTyrAsnAlaLysAlaGlyLeuCysGlnThrPheValTyrGly
ATCAGATACTTCTACAACGCCAAGGCTGGTTTGTGTCAAACTTTCGTTTACGGT
TAGTCTATGAAGATGTTGCGGTTCCGACCAAACACAGTTTGAAAGCAAATGCCA

GlyCysArgAlaLysSerAsnAsnPheLysSerAlaGluAspCysMetArgThr
GGCTGCAGAGCTAAGTCCAACAACTTCAAGTCTGCTGAAGACTGCATGAGAACT
CCGACGTCTCGATTCAGGTTGTTGAAGTTCAGACGACTTCTGACGTACTCTTGA
 
CysGlyGlyAla
TGTGGTGGTGCC
ACACCACCACGG ,
 oder eine funktionell äquivalente Sequenz.

15. Ein Hefestamm, der mit einem Vektor nach Anspruch 10 transformiert ist.

## Revendications

1. Procédé de production d'aprotinine ou de ses homologues dans une levure, comprenant la culture dans un milieu de culture convenable d'une souche de levure contenant un vecteur d'expression réplicable, le vecteur comprenant une séquence d'ADN codant pour l'aprotinine ou un homologue d'aprotinine fusionnée avec une séquence d'ADN codant pour un peptide signal et de tête assurant la sécrétion de l'aprotinine ou de l'homologue d'aprotinine, et la récupération de l'aprotinine ou de l'homologue d'aprotinine sécrété à partir du milieu de culture.

2. Procédé selon la revendication 1, dans lequel la séquence d'ADN code pour l'aprotinine(3-58).

3. Procédé selon la revendication 2, dans lequel la séquence d'ADN a la séquence suivante: 
AspPheCysLeuGluProProTyrThrGlyProCysLysAlaArgIleIleArg
GATTTCTGTTTGGAACCTCCATACACTGGTCCATGTAAAGCTAGAATCATCAGA
CTAAAGACAAACCTTGGAGGTATGTGACCAGGTACATTTCGATCTTAGTAGTCT

TyrPheTyrAsnAlaLysAlaGlyLeuCysGlnThrPheValTyrGlyGlyCys
TACTTCTACAACGCCAAGGCTGGTTTGTGTCAAACTTTCGTTTACGGTGGCTGC
ATGAAGATGTTGCGGTTCCGACCAAACACAGTTTGAAAGCAAATGCCACCGACG

ArgAlaLysArgAsnAsnPheLysSerAlaGluAspCysMetArgThrCysGly
AGAGCTAAGAGAAACAACTTCAAGTCTGCTGAAGACTGCATGAGAACTTGTGGT
TCTCGATTCTCTTTGTTGAAGTTCAGACGACTTCTGACGTACTCTTGAACACCA

GlyAla
GGTGCC
CCACGG
 ou une séquence fonctionnellement équivalente.

4. Procédé selon la revendication 1, dans lequel la séquence d'ADN code pour l'aprotinine(3-58,42 Ser).

5. Procédé selon la revendication 4, dans lequel la séquence d'ADN a la séquence suivante: 
AspPheCysLeuGluProProTyrThrGlyProCysLysAlaArgIleIleArg
GATTTCTGTTTGGAACCTCCATACACTGGTCCATGTAAAGCTAGAATCATCAGA
CTAAAGACAAACCTTGGAGGTATGTGACCAGGTACATTTCGATCTTAGTAGTCT

TyrPheTyrAsnAlaLysAlaGlyLeuCysGlnThrPheValTyrGlyGlyCys
TACTTCTACAACGCCAAGGCTGGTTTGTGTCAAACTTTCGTTTACGGTGGCTGC
ATGAAGATGTTGCGGTTCCGACCAAACACAGTTTGAAAGCAAATGCCACCGACG

ArgAlaLysSerAsnAsnPheLysSerAlaGluAspCysMetArgThrCysGly
AGAGCTAAGTCCAACAACTTCAAGTCTGCTGAAGACTGCATGAGAACTTGTGGT
TCTCGATTCAGGTTGTTGAAGTTCAGACGACTTCTGACGTACTCTTGAACACCA

GlyAla
GGTGCC
CCACGG
 ou une séquence fonctionnellement équivalente.

6. Procédé selon la revendication 1, dans lequel la séquence d'ADN code pour l'aprotinine(1-58).

7. Procédé selon la revendication 6, dans lequel la séquence d'ADN a la séquence suivante: 

AGGCCTGATTTCTGTTTGGAACCTCCATACACTGGTCCATGTAAAGCTAGAATC
TCCGGACTAAAGACAAACCTTGGAGGTATGTGACCAGGTACATTTCGATCTTAG


ATCAGATACTTCTACAACGCCAAGGCTGGTTTGTGTCAAACTTTCGTTTACGGT
TAGTCTATGAAGATGTTGCGGTTCCGACCAAACACAGTTTGAAAGCAAATGCCA


GGCTGCAGAGCTAAGAGAAACAACTTCAAGTCTGCTGAAGACTGCATGAGAACT
CCGACGTCTCGATTCTCTTTGTTGAAGTTCAGACGACTTCTGACGTACTCTTGA


TGTGGTGGTGCC
ACACCACCACGG
 ou une séquence fonctionnellement équivalente.

8. Procédé selon la revendication 1, dans lequel la séquence d'ADN code pour l'aprotinine(1-58,42 Ser).

9. Procédé selon la revendication 8, dans lequel la séquence d'ADN a la séquence suivante: 
ArgProAspPheCysLeuGluProProTyrThrGlyProCysLysAlaArgIle
AGGCCTGATTTCTGTTTGGAACCTCCATACACTGGTCCATGTAAAGCTAGAATC
TCCGGACTAAAGACAAACCTTGGAGGTATGTGACCAGGTACATTTCGATCTTAG

IleArgTyrPheTyrAsnAlaLysAlaGlyLeuCysGlnThrPheValTyrGly
ATCAGATACTTCTACAACGCCAAGGCTGGTTTGTGTCAAACTTTCGTTTACGGT
TAGTCTATGAAGATGTTGCGGTTCCGACCAAACACAGTTTGAAAGCAAATGCCA

GlyCysArgAlaLysSerAsnAsnPheLysSerAlaGluAspCysMetArgThr
GGCTGCAGAGCTAAGTCCAACAACTTCAAGTCTGCTGAAGACTGCATGAGAACT
CCGACGTCTCGATTCAGGTTGTTGAAGTTCAGACGACTTCTGACGTACTCTTGA

CysGlyGlyAla
TGTGGTGGTGCC
ACACCACCACGG
 ou une séquence fonctionnellement équivalente.

10. Vecteur capable de se répliquer dans une levure et comprenant une séquence d'ADN codant pour l'aprotinine ou un homologue d'aprotinine fusionnée a une séquence d'ADN codant pour un peptide signal et de tête assurant la sécrétion de l'aprotinine ou de l'homologue d'aprotinine.

11. Vecteur selon la revendication 10, dans lequel la séquence d'ADN code pour l'aprotinine(3-58) et a la séquence suivante: 
AspPheCysLeuGluProProTyrThrGlyProCysLysAlaArgIleIleArg
GATTTCTGTTTGGAACCTCCATACACTGGTCCATGTAAAGCTAGAATCATCAGA
CTAAAGACAAACCTTGGAGGTATGTGACCAGGTACATTTCGATCTTAGTAGTCT

TyrPheTyrAsnAlaLysAlaGlyLeuCysGlnThrPheValTyrGlyGlyCys
TACTTCTACAACGCCAAGGCTGGTTTGTGTCAAACTTTCGTTTACGGTGGCTGC
ATGAAGATGTTGCGGTTCCGACCAAACACAGTTTGAAAGCAAATGCCACCGACG
 
ArgAlaLysArgAsnAsnPheLysSerAlaGluAspCysMetArgThrCysGly
AGAGCTAAGAGAAACAACTTCAAGTCTGCTGAAGACTGCATGAGAACTTGTGGT
TCTCGATTCTCTTTGTTGAAGTTCAGACGACTTCTGACGTACTCTTGAACACCA

GlyAla
GGTGCC
CCACGG
 ou une séquence fonctionnellement équivalente.

12. Vecteur selon la revendication 10, dans lequel la séquence d'ADN code pour l'aprotinine(3-58,42 Ser) et a la séquence suivante: 
AspPheCysLeuGluProProTyrThrGlyProCysLysAlaArgIleIleArg
GATTTCTGTTTGGAACCTCCATACACTGGTCCATGTAAAGCTAGAATCATCAGA
CTAAAGACAAACCTTGGAGGTATGTGACCAGGTACATTTCGATCTTAGTAGTCT

TyrPheTyrAsnAlaLysAlaGlyLeuCysGlnThrPheValTyrGlyGlyCys
TACTTCTACAACGCCAAGGCTGGTTTGTGTCAAACTTTCGTTTACGGTGGCTGC
ATGAAGATGTTGCGGTTCCGACCAAACACAGTTTGAAAGCAAATGCCACCGACG

ArgAlaLysSerAsnAsnPheLysSerAlaGluAspCysMetArgThrCysGly
AGAGCTAAGTCCAACAACTTCAAGTCTGCTGAAGACTGCATGAGAACTTGTGGT
TCTCGATTCAGGTTGTTGAAGTTCAGACGACTTCTGACGTACTCTTGAACACCA

GlyAla
GGTGCC
CCACGG
 ou une séquence fonctionnellement équivalente.

13. Vecteur selon la revendication 10, dans lequel la séquence d'ADN code pour l'aprotinine(1-58) et a la séquence suivante: 

AGGCCTGATTTCTGTTTGGAACCTCCATACACTGGTCCATGTAAAGCTAGAATC
TCCGGACTAAAGACAAACCTTGGAGGTATGTGACCAGGTACATTTCGATCTTAG


ATCAGATACTTCTACAACGCCAAGGCTGGTTTGTGTCAAACTTTCGTTTACGGT
TAGTCTATGAAGATGTTGCGGTTCCGACCAAACACAGTTTGAAAGCAAATGCCA
 

GGCTGCAGAGCTAAGAGAAACAACTTCAAGTCTGCTGAAGACTGCATGAGAACT
CCGACGTCTCGATTCTCTTTGTTGAAGTTCAGACGACTTCTGACGTACTCTTGA


TGTGGTGGTGCC
ACACCACCACGG
 ou une séquence fonctionnellement équivalente.

14. Vecteur selon la revendication 10, dans lequel la séquence d'ADN code pour l'aprotinine(1-58,42 Ser) et a la séquence suivante: 
ArgProAspPheCysLeuGluProProTyrThrGlyProCysLysAlaArgIle
AGGCCTGATTTCTGTTTGGAACCTCCATACACTGGTCCATGTAAAGCTAGAATC
TCCGGACTAAAGACAAACCTTGGAGGTATGTGACCAGGTACATTTCGATCTTAG

IleArgTyrPheTyrAsnAlaLysAlaGlyLeuCysGlnThrPheValTyrGly
ATCAGATACTTCTACAACGCCAAGGCTGGTTTGTGTCAAACTTTCGTTTACGGT
TAGTCTATGAAGATGTTGCGGTTCCGACCAAACACAGTTTGAAAGCAAATGCCA

GlyCysArgAlaLysSerAsnAsnPheLysSerAlaGluAspCysMetArgThr
GGCTGCAGAGCTAAGTCCAACAACTTCAAGTCTGCTGAAGACTGCATGAGAACT
CCGACGTCTCGATTCAGGTTGTTGAAGTTCAGACGACTTCTGACGTACTCTTGA

CysGlyGlyAla
TGTGGTGGTGCC
ACACCACCACGG
 ou une séquence fonctionnellement équivalente.

15. Souche de levure transformée avec un vecteur selon la revendication 10.
